# EUROPEAN PATENT APPLICATION

(11) **EP 3 900 845 A1**
(43) Date of publication of application: **27.10.2021**
(21) Application number: 20170613.2
(22) Date of filing: 21.04.2020
(51) Int. Cl.: B06B 1/02, A61B 8/12, G01S 7/52, G01S 15/89

(54) **ULTRASOUND DEVICE**

(71) Applicant: Koninklijke Philips N.V., 5656 AG Eindhoven (NL)
(72) Inventor: HENNEKEN, Vincent Adrianus, 5656 AE Eindhoven (NL); DEKKER, Ronald, 5656 AE Eindhoven (NL); VAN RENS, Antonia Cornelia, 5656 AE Eindhoven (NL); LOUWERSE, Marcus Cornelis, 5656 AE Eindhoven (NL)
(74) Representative: Philips Intellectual Property & Standards

(57) **Abstract**

An ultrasound device has an ultrasound scanner unit at a distal portion of a shaft. The ultrasound scanner unit has ultrasound transducers and capacitors, each comprising a bottom electrode provided over a substrate, a region over the bottom electrode, a dielectric layer over (and preferably also beneath) the region and a top electrode over the dielectric layer. For the ultrasound transducers, the region is a cavity formed by removing a sacrificial layer. For the capacitors, the region may be the sacrificial layer. Capacitors can thus be formed integrally with the cMUT elements.

## Description

### FIELD OF THE INVENTION

This invention relates to devices, which incorporate ultrasound imaging at the tip of a shaft.

### BACKGROUND OF THE INVENTION

Intravascular ultrasound (IVUS) imaging catheters and intra-cardiac ultrasound (ICE) catheters, are well known. They typically incorporate piezoelectric ultrasound elements at the catheter tip, for performing localized imaging.

The catheter shaft needs to be flexible, to enable the catheter to be steered within the blood vessels and within the heart. However, the ultrasound head needs to be formed as a rigid structure, and it is typically formed at a rigid tip part of the catheter.

There is a desire to make use of capacitive micro-machined ultrasound transducers (cMUTs). cMUT devices using such transducers are becoming increasingly popular because they can offer excellent bandwidth and acoustic impedance characteristics, which makes them preferable over piezoelectric transducers. Vibration of the membrane of the cMUT can be induced electrically.

In cMUT imaging devices with extreme size restrictions, such as in-body devices, it is very difficult to implement discrete high voltage capacitors as well as high quality electrical wires.

There is therefore a need to improve the provision of capacitors with cMUT devices.

### SUMMARY OF THE INVENTION

The invention is defined by the claims.

According to examples in accordance with an aspect of the invention, there is provided an ultrasound device comprising:
a shaft;
an ultrasound scanner unit or an ultrasound head at a distal portion of the shaft comprising an array of cMUT ultrasound transducers and one or more capacitors,
wherein the ultrasound transducers each comprise:
   a bottom electrode provided over a substrate;
   a cavity over the bottom electrode;
   a dielectric layer over the cavity; and
   a top electrode over the dielectric layer,
wherein the one or more capacitors comprise:
   a bottom electrode provided over the substrate and formed from the same layer as the ultrasound transducer bottom electrodes;
   a dielectric layer formed from the same layer as the ultrasound transducer dielectric layer; and
   a top electrode over the dielectric layer and formed from the same material as the ultrasound transducer top electrodes.

In this way, capacitors such as high voltage decoupling capacitors, may be integrated with the same dielectric and electrode (metal) layers as the cMUT elements. The capacitor values are for example in the sub-nF range. The term "cMUT element" is used to denote a single cMUT cell, i.e. a single cMUT transducer.

cMUT devices enable an array of transducers to be formed, for example so that electronic beam-forming in 2D or 3D becomes possible. The transducers are then produced in arrays with transducer element sizes of e.g. λ/2 where λ is the wavelength of the used ultrasound frequency in body tissue.

A large number of transducers in the array is desired, for example disposed around a perimeter of the catheter in order to obtain high-resolution images given a certain ultrasound frequency.

By way of example the number of transducer elements is greater than or equal to 100, the scanner diameter is less than or equal to 1 mm, the scanner length is less than or equal to 5 mm, and inner lumen diameter is around 0.5 mm.

cMUT array devices require local integrated circuitry (in particular ASICs) to enable operation of the cMUT elements. For example, high voltage pulses of a pulser circuit and a high voltage DC bias are generated by a probe circuit, which is typically an application specific integrated circuit (ASIC) which is located within the ultrasound probe, i.e. at the probe location. The ASIC facilitates both transmission and reception modes of the device. In reception mode, changes in the membrane position cause changes in electrical capacitance, which can be registered electronically. In transmission mode, applying an electrical signal causes vibration of the membrane. Besides the ASICs, several capacitors are also needed to enable a stable power supply to the ASIC and cMUT. By making the capacitors, such as high voltage decoupling capacitors, from the same structural layers as the cMUT tranducers, the size of capacitors can be miniaturized with respect to using other capacitors, since the cpacitors are readlily integrated and no additional electrical connections are needed to be made. In cMUT imaging devices with extreme size restrictions, such as in-body devices, it is very difficult to implement discrete high voltage capacitors as well as high quality electrical wires. Even with high quality wires, the non-zero characteristic impedance of the wires impacts the voltage stability due to the limited space.

A sacrificial layer is used to form the cavity of the cMUT elements, such as a metal sacrificial layer. This layer may remain in place in the capacitor structure, or it may be removed from the area in which the capacitors are formed.

When the sacrificial layer remains in place for the capacitor structure, the device may further comprise a connection terminal to the sacrificial layer of the one or more capacitors. The layer structure may form two capacitors in series in a stack. By enabling electrical connection to the central terminal, the capacitors can instead be electrically connected in parallel, resulting in a four times increase in capacitance for the same physical structure. The two capacitors may also be used as separate, independent capacitors, sharing one common voltage, such as ground, at the central terminal.

One electrode of each ultrasound transducer (e.g. the bottom electrode) for example connects to a ground terminal and the other electrode of each ultrasound transducer (e.g. the top electrode) connects to a control terminal. Thus a control terminal signal is used to control deformation of the top electrode.

Each ultrasound transducer is for example part of a transducer circuit, which comprises a bias terminal for applying a bias voltage to the ultrasound transducer and a stimulus terminal for applying a drive signal to the ultrasound transducer. The bias is for example used to drive the cMUT element into a collapsed mode. This is a known effective way to operate cMUT elements.

The transducer circuit may comprise a bias-T circuit comprising, for each ultrasound transducer:
a resistor between the bias terminal and the control terminal; and
the capacitor between the stimulus terminal and the control terminal.

This bias-T circuit is known (for example from US 2018/164418). The capacitors provide a reduction in cross talk between the cMUT elements. Such crosstalk is for example likely when a common capacitor is used for stabilization of the cMUT bias voltage. The resistors function as buffer elements to prevent a low-impedance short between the cMUT element bias node and the counter electrode in the case of a cMUT element short circuit. In this way, failure of an individual cell will not cause a breakdown of the whole cMUT array nor a breakdown of the driving electronics.

In another use of the capacitor, the bias terminal and the stimulus terminal comprise a shared input, and each ultrasound transducer is part of an amplifier circuit, comprising an amplifier and a high pass filter, wherein a first input terminal of the amplifier connects to the shared input through the ultrasound transducer, and a second input terminal of the amplifier connects to the shared input through a high pass filter which comprises the capacitor and a resistor. The filter suppresses high frequency disturbances. The use of a shared input reduces the number of wire connections which need to be made.

In another use of the capacitor, the at least one capacitor comprises a first capacitor for the bias voltage and second capacitor for a high voltage supply signal. Thus, separate decoupling capacitors may be provided.

The device may comprise:
a first flexible carrier region on which the ultrasound transducers are mounted;
a set of integrated circuits for controlling the ultrasound transducers, the integrated circuits being located proximally of the ultrasound transducers and connected at a distal end to ultrasound transducers of the array,
wherein the capacitors are formed on the first flexible carrier region.

The ultrasound transducers may for example be wrapped around the shaft by means of the first flexible carrier region.

The electrical connection between the integrated circuits and the ultrasound head is for example implemented with a flex-to-rigid interconnection.

The device may further comprise:
a second flexible carrier region which defines an interconnect ring located proximally of the set of integrated circuits and connected to a proximal end of the integrated circuits; and
a cable attached to the interconnect ring,
wherein the capacitors are formed on the first and second flexible carrier regions.

This example makes use of an interconnect ring between the cable and the set of integrated circuits. It enables signal lines to be redistributed to the integrated circuits at the proximal side of the integrated circuits. The interconnect ring enables extra electrical connections between the integrated circuits as well as a bus connection from the proximal (system) side using a finite number of wires in the cable.

The interconnect ring also provides the improved assembly stability of the ultrasound head or the ultrasound array, because the integrated circuits (which are typically thin finger-shaped components so that they can wrap around the shaft) are supported at both ends, thereby restricting uncontrolled motions of individual circuits during the assembly process.

The flexible carrier regions may each thus be realized in the Flex-to-Rigid (F2R) manufacturing platform, along with the other parts of the ultrasound transducer component. For example, the F2R manufacturing platform may define silicon islands which function as support dies for the integrated circuits as well as the flexible regions and the flexible parts of the connections between those silicon islands and the ultrasound head and interconnect ring.

The integrated circuits for example comprise a master ASIC and a set of slave ASICs. The cable then connects to the master ASIC and the interconnect ring provides connections between the master ASIC and the slave ASICs.

The integrated circuits are for example each flip chip bonded to a respective rigid base, such as a silicon island of the F2R platform. The base is then coupled to the interconnect ring on one side and the ultrasound head at the other side.

The set of integrated circuits for example is arranged as an annulus around an elongate axis of the shaft. This provides an optimum use of space.

The array of ultrasound transducers is for example arranged as a one dimensional array around an elongate axis of the shaft. This is a known arrangement of the transducers, to enable all around imaging of the area around the shaft.

The ultrasound device for example comprises a catheter, and the shaft is the catheter shaft.

### BRIEF DESCRIPTION OF THE DRAWINGS

Examples of the invention will now be described in detail with reference to the accompanying drawings, in which:
Figure 1 shows a schematic drawing of an example ultrasound imaging catheter;
Figure 2 schematically depicts a typical cMUT element of an ultrasound system operable in a collapsed mode;
Figures 3 and 4 depict operating principles of such a cMUT element;
Figure 5 shows the basic cMUT structure;
Figure 6 shows a first capacitor layout using the same structure;
Figure 7 shows an alternative capacitor layout;
Figure 8 shows the cMUT element within a transducer circuit in the form of a bias-T circuit;
Figure 9 shows the cMUT element within a transducer circuit in the form of an amplifier circuit;
Figure 10 shows a possible design before being wrapped around the catheter core;
Figure 11 shows the arrangement of Figure 10 provided around the catheter core;
Figure 12 shows a variant in which integrated circuits comprise a master ASIC 12a and a set of slave ASICs; and
Figure 13 shows how this structure may be used for integrating two types of capacitor.

### DETAILED DESCRIPTION OF THE EMBODIMENTS

The invention provides an ultrasound device having an ultrasound head at a distal end of a shaft. The ultrasound head, or ultrasound scanner unit, has ultrasound transducers and capacitor, each comprising a bottom electrode provided over a substrate, a region over the bottom electrode, a dielectric layer over (and typically also beneath) the region and a top electrode over the top dielectric layer. For the ultrasound transducers, the region is a cavity formed by removing a sacrificial layer. For the capacitors, the region may be the sacrificial layer or else no sacrificial layer may be present in the capacitor structure. Capacitors can thus be formed integrally with the cMUT elements.

The invention will be described with reference to ultrasound imaging at the tip of a catheter.

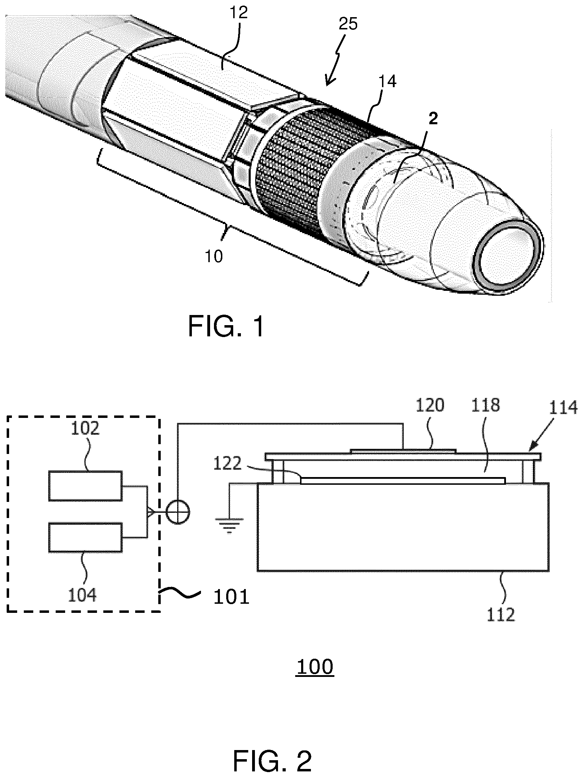

Figure 1 shows a schematic drawing of an example ultrasound imaging catheter.

The catheter has an outer diameter of approximately 1 mm. Internally, there is a shaft or a core 2 that may be optionally hollow, for example for guiding the catheter along a guidewire.

The catheter comprises an imaging assembly or imaging core 10 having an array of integrated circuits 12. These are control ASICs mounted by flip-chip binding to supports, such as support dies.

These support dies are formed by the substrate of the F2R interconnect technology, as described below.

The integrated circuits have a length in the direction parallel to the length direction of the catheter and a width in the perpendicular direction. They are arranged around the catheter, with their width directions aligned around the catheter circumference.

The imaging core further comprises an array 14 of ultrasound transducers. The transducers basically comprise an acoustic generator and receiver in the form of a cMUT element. cMUT elements can be made by semiconductor processes and in particular the same processes used to produce the application specific integrated circuits (ASICs) needed by an ultrasound probe such as a CMOS process.

cMUT elements are tiny diaphragm-like devices with electrodes that convert the sound vibration of a received ultrasound signal into a modulated capacitance. Many such individual cMUT elements can be connected together and operated in a synchronized manner as a single transducer element. For example, four to twenty four cMUT elements can be coupled together to function in synchronized manner (e.g. phase shifted driven or simultaneously driven) as a single transducer element.

The manufacturing of cMUT-based ultrasound systems is therefore more cost-effective compared to PZT-based systems. Moreover, due to the materials used in such semiconductor processes, the cMUT elements exhibit much improved acoustic impedance matching to water and biological tissue, which obviates the need for (multiple) matching layers and yields an improved effective bandwidth.

cMUT elements in particular are able to function over a broad bandwidth, enable high resolution and high sensitivity imaging, and produce a large pressure output so that acoustic signals from a large depth can be received at ultrasonic frequencies.

Figure 2 shows a known design of cMUT element 100 (a so-called cell) for use in an ultrasound system and a drive arrangement 101. The cMUT element 100 comprises a flexible membrane or diaphragm 114 suspended above a silicon substrate 112 with a gap or cavity 118 there between. A first electrode 122 is located on the floor of the cell on the upper surface of the substrate 112 in this example. A second electrode 120 is located on the diaphragm 114 and moves with the diaphragm. The flexible membrane and the first electrode are separated by the cavity 118. In the example shown, the two electrodes are circular.

A dielectric (not shown) is provided on the substrate 112 and underneath the top (second) electrode 120. The diaphragm may instead be or function as a dielectric layer.

Preferably, there are two dielectrics which may be equal in composition and thickness, but may be also asymmetric (different materials and thicknesses).

The membrane layer 114 is fixed relative to the top face of the substrate layer 112 and configured and dimensioned so as to define a spherical or cylindrical cavity 118 between the membrane layer 114 and the substrate layer 112.

Other realizations of the electrode 120 design can be considered, such as electrode 120 may be embedded in the membrane 114 or it may be deposited on the membrane 114 as an additional layer.

The first electrode may be directly exposed to the gap 118 or separated from the gap 118 by an electrically insulating layer or film to prevent a short-circuit between the second electrode 120 and the first electrode 122.

In Figure 2 the first electrode 122 is grounded by way of example. Other arrangements, e.g. a grounded second electrode 120 or both second electrode 120 and first electrode 122 floating are of course equally feasible.

The electrodes of the cMUT element 100 provide the capacitive plates of the device and the gap 118 is the main dielectric between the plates of the capacitor. When the diaphragm vibrates, the changing dimension of the dielectric gap between the plates provides a changing capacitance which is sensed as the response of the cMUT element 100 to a received acoustic echo.

The spacing between the electrodes is controlled by applying a static voltage, e.g. a DC bias voltage, to the electrodes with a voltage supply 101. The voltage supply 101 may optionally comprise separate stages 102, 104 for providing the DC and AC or stimulus components respectively of the drive voltage of the cMUT elements 100, e.g. in transmission mode. The first stage 102 may be adapted to generate the static (DC) voltage component and the second stage 104 may be adapted to generate an alternating variable drive or stimulus voltage component having a set alternating frequency, which signal typically is the difference between the overall drive voltage and the aforementioned static component thereof.

The static or bias component of the applied drive voltage preferably meets or exceeds the threshold voltage for forcing the cMUT element 100 into its collapsed state. This has the advantage that the first stage 102 may include relatively large capacitors, e.g. smoothing capacitors, in order to generate a particularly low-noise static component of the overall voltage, which static component typically dominates the overall voltage such that the noise characteristics of the overall voltage signal will be dominated by the noise characteristics of this static component.

Bby applying a static voltage above a certain threshold, the cMUT element 100 is forced into a collapsed state in which the membrane 114 collapses onto the substrate 112. This threshold value may depend on the exact design of the cMUT element 100 and is defined as the DC bias voltage, also called as the collapse voltage, at which the membrane 114 sticks to (contacts) the cell floor through the force due to the electric field between the electrodes. The amount (area) of contact between the membrane 114 and the substrate 112 is dependent on the applied bias voltage.

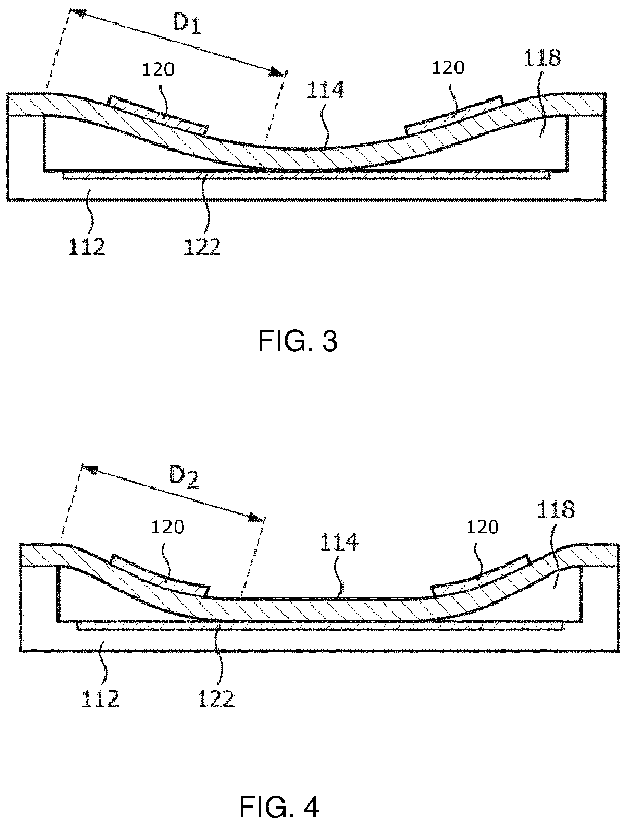

Increasing the contact area between the membrane 114 and the substrate 112 increases the resonant frequency of the membrane 114, as will be explained in more detail with the aid of Figure 3 and Figure 4.

The frequency response of a collapsed mode cMUT element 100 may be varied by adjusting the DC bias voltage applied to the cMUT electrodes after collapse. As a result, the resonant frequency of the cMUT element increases as a higher DC bias voltage is applied to the electrodes.

The principles behind this phenomenon are illustrated in Figures 3 and 4. The cross-sectional views of Figures 3 and 4 illustrate this one-dimensionally by the distances D1 and D2 between the outer support of the membrane 114 and the point where the membrane begins to touch the floor through the cavity 118 in each illustration. It can be seen that the distance D1 is a relatively long distance in Figure 3 when a relatively low bias voltage is applied, whereas the distance D2 in Figure 4 is a much shorter distance due to a higher bias voltage being applied. These distances can be compared to long and short strings which are held by the ends and then plucked. The long, relaxed string will vibrate at a much lower frequency when plucked than will the shorter, tighter string. Analogously, the resonant frequency of the cMUT element in Figure 3 will be lower than the resonant frequency of the cMUT element in Figure 4 which is subject to the higher bias voltage.

Thus, a typical cMUT design comprises a flexible membrane or diaphragm suspended above a silicon substrate with a gap or cavity between them. The gap is caused by removing a sacrificial layer during manufacture. A first electrode is located on the floor of the cell on the upper surface of the substrate and a second electrode is located on the diaphragm and moves with the diaphragm.

The cMUT elements are formed on a flexible carrier so they can be wrapped around the periphery of the core 2 of the catheter, as shown in Figure 1. The integrated circuits can similarly be wrapped around the catheter because despite being rigid, there are formed as separate fingers. The connections between the integrated circuits 12 and the array 14 is by means of flexible to rigid interconnects 25, preferably using the so-called Flex-to-Rigid (F2R) interconnection system.

Flex-to-rigid (F2R) is a technology platform for the fabrication of miniature partially flexible sensor systems for minimally invasive medical instruments. Because of the flexibility, these sensor systems can be wrapped around cylindrical medical instruments such as catheters or guidewires. F2R is a postprocessing technology whereby flexible interconnects, consisting of a metal routing layer sandwiched between two layers of flexible polyimide, are fabricated on a silicon wafer. This is followed by a separation step whereby individual elements are singulated by Deep Reactive Ion Etching (DRIE) and Reactive Ion Etching (RIE). The F2R fabrication is based on standard IC fabrication technology, which allows the devices to scale down so that all kind of sensing functionalities and electronics can be integrated into the tip of a catheter.

The invention is based on the use of the cMUT layer stack to create parallel plate capacitors. This may for example be achieved by forming a region in which the sacrificial metal layer between the electrodes is not removed. Alternatively, the sacrificial layer may not be present at all in the regions where the capacitors are formed. In this way, the capacitive density of the capacitors is maximized and no sound is produced during use of these capacitors. High voltage capacitors can be implemented at areas where no acoustic cMUT elements are intended, such as at or near and under the bond pad locations.

The cMUT structure is well suited for integration of capacitors because the dielectric layers are specifically tuned to the voltage levels needed for the cMUT elements and therefore also for the capacitors. The optionally remaining sacrificial metal layer is electrically invisible to the performance of the capacitors.

In one of the embodiments the capacitor comprises: a bottom electrode provided over the substrate and formed from the same material as the ultrasound transducer bottom electrodes; a dielectric layer formed from the same material as the ultrasound transducer dielectric layer; and a top electrode over the dielectric layer and formed from the same material as the ultrasound transducer top electrodes. Furthermore, the capacitor may comprise: a bottom electrode provided over the substrate and formed from the same layer as the ultrasound transducer bottom electrodes; a dielectric layer formed from the same layer as the ultrasound transducer dielectric layer; and a top electrode over the dielectric layer and formed from the same layer as the ultrasound transducer top electrodes. In case one of the layers of the capacitor needs to be of a thickness different from the corresponding layer that is used for the cMUT transducer, then that can be obtained by various extents of shadow masking during deposition of the specific layer.

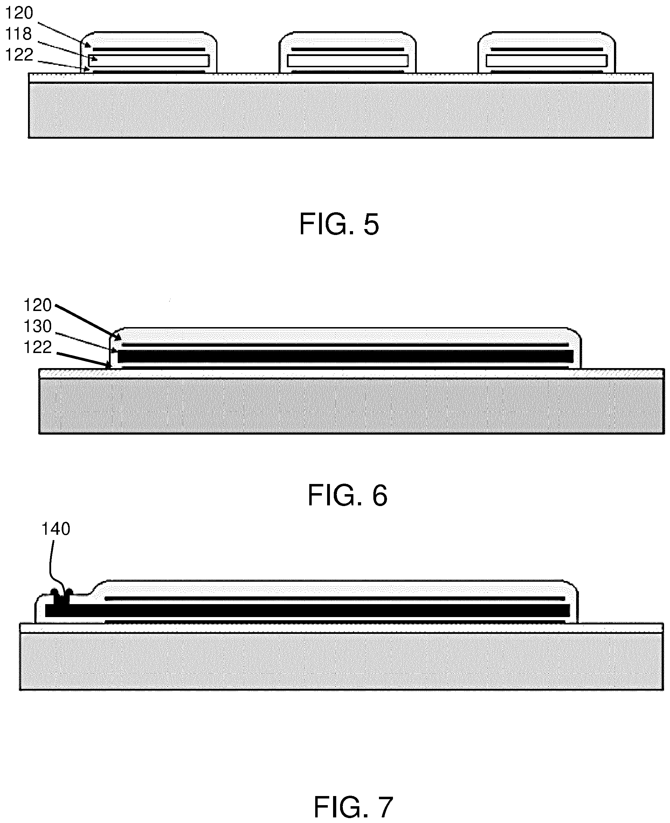

Figure 5 shows an embodiment of a cMUT structure, showing the top electrode 120, bottom electrode 122 and cavity 118, but wherein the bottom electrode is covered by a first dielectric layer, that is separated from the membrane 114 by the cavity 118, and over the membrane the top electrode 120 is deposited and patterned, which optionally can be covered by a further dielectric layer.

Figure 6 shows a capacitor using the same structure. A larger unit is formed to create the desired capacitance.

The cMUT membrane diameter depends on the ultrasound frequency aimed for; the higher the frequency, the smaller the diameter. For example, for a 30-40 MHz ultrasound frequency the membrane diameter is in the order of 25 µm. For IVUS catheters, a typical element consists of multiple cMUT membranes connected in a line, with a line dimensions for example of 25-30 µm x 600 µm.

A larger capacitor corresponds to a larger capacitance, so that as much available silicon area on the transducer component is used as possible for the capacitors. Typically, the capacitors can be almost as large as the available silicon dies, e.g. for ASIC connections. They are not necessarily circular.

In Figure 6, dielectric layers are shown above the bottom electrode and beneath the top electrode. These dielectric layers may have the same thickness but this is not essential. Indeed, as a minimum, one dielectric layer is needed for example at the top electrode.

In the configuration of Figure 6, the sacrificial metal layer 130 is electrically floating. The sacrificial metal layer may instead be removed at the capacitor structures (or not deposited), for example so that the two dielectric layers are directly over each other, with no cavity. In a first example, the sacrificial layer 130 is left in place and forms a central, floating, electrode. This structure effectively creates two capacitors of capacitance C in series, hence a total capacitance of C/2. The capacitance C is the capacitance between top and central electrode, and between the central electrode and bottom electrode. For a given breakdown voltage Vbr of each of the capacitors, the effective breakdown voltage of the structure is 2Vbr.

The machining the cMUT element of Figure 5 together with the capacitor of Figure 6 provides an integrated way of obtaining the cMUT element with the capacitor, as the structural and functional layers of the cMUT layers are identical with those of the capacitor, with the exception that the sacrificial layer 130 is removed from the cMUT element, resulting in cavity 118, whereas for the capacitor may be left in place, or alternatively may not be deposited, thereby the first dielectric layer covering the bottom electrode 122 and the second dielectric layer (forming also the membrane of the cMUT) will be deposited upon each other, which then will be covered by the top electrode 120 layer for both the cMUT element and the capacitor. Further option is to cover the top electrode 120 with a dielectric layer.

Figure 7 shows an alternative in which the sacrificial metal is left in place between the top and bottom electrodes, and is also electrically connected to a connection terminal 140.

By connecting the top and bottom electrodes together, they define one terminal of a capacitor arrangement and the central connection terminal defines the other terminal. The capacitor arrangement thereby comprises the two capacitors of capacitance C in parallel hence a total capacitance of 2C (a four fold increase). The total breakdown voltage reduces to Vbr.

This requires one additional processing mask step.

The top and bottom electrodes do not need to be connected to each other, so the top and bottom capacitors could instead be used as independent capacitors.

There are various possible uses for the capacitors that are formed.

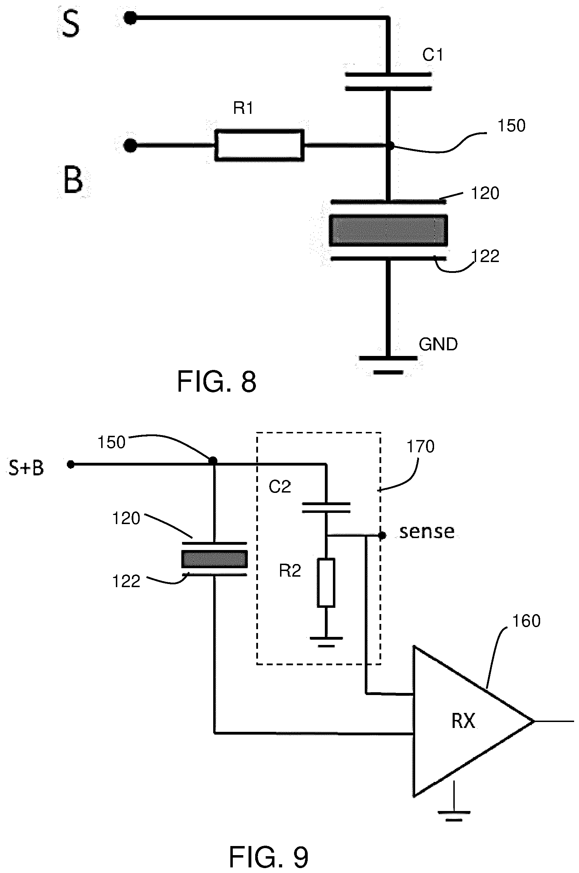

Figure 8 shows the cMUT element within a transducer circuit in the form of a bias-T circuit. The bottom electrode 122 of the ultrasound transducer is shown connected to a ground terminal and the top electrode 120 connects to a control terminal 150. Thus a control terminal signal is used to control deformation of the top electrode.

The bias-T circuit comprises a resistor R1 between a bias terminal B and the control terminal 150 and the capacitor C1 between a stimulus terminal S and the control terminal 150.

The capacitor in this circuit provides a reduction in cross talk between the cMUT elements. Such crosstalk is for example likely when a common capacitor is used for stabilization of the cMUT bias voltage. The resistors function as buffer elements to prevent a low-impedance short between the cMUT element control terminal and the counter electrode in the case of a cMUT element short circuit. In this way, failure of an individual cell will not cause a breakdown of the whole cMUT array nor a breakdown of the driving electronics.

A high-voltage coupling capacitor is needed for the bias-T circuit as well as a high value resistor. The bias-T network combines the AC-component of the cMUT stimulus signal with the DC-component which forms the bias voltage of the cMUT.

Figure 9 shows another use of the capacitor. The cMUT element is again within a transducer circuit in the form of an amplifier circuit. The bias terminal B and the stimulus terminal S comprise a shared input. The role of the capacitor is to sense the AC signal of a combined bias voltage and stimulus cMUT signal. A combined cMUT signal is attractive in cases where the number of wires in the catheter is severely restricted due to area restrictions. Sensing the AC-component of the cMUT driving signal may be needed for signaling purposes and to suppress high-frequency disturbances that can happen on the 'S+B' node.

The circuit comprises an amplifier 160 and a high pass filter 170. A first input terminal of the amplifier connects to the shared input through the ultrasound transducer, and a second input terminal of the amplifier connects to the shared input through the high pass filter 170. The high pas filter comprises the capacitor C2 and a resistor R2. The filter suppresses high frequency disturbances. In this case, the amplifier circuit is set as a transimpedance amplifier, of which the negative input terminal (virtual ground) is connected to the cMUT element and the positive input terminal (high impedance) is connected to the sense node at the output of the high pass filter.

It may also be desired to use separate high voltage capacitors to stabilize the cMUT bias voltage as well as the high voltage supply (VDDH) to the connected ASIC independently.

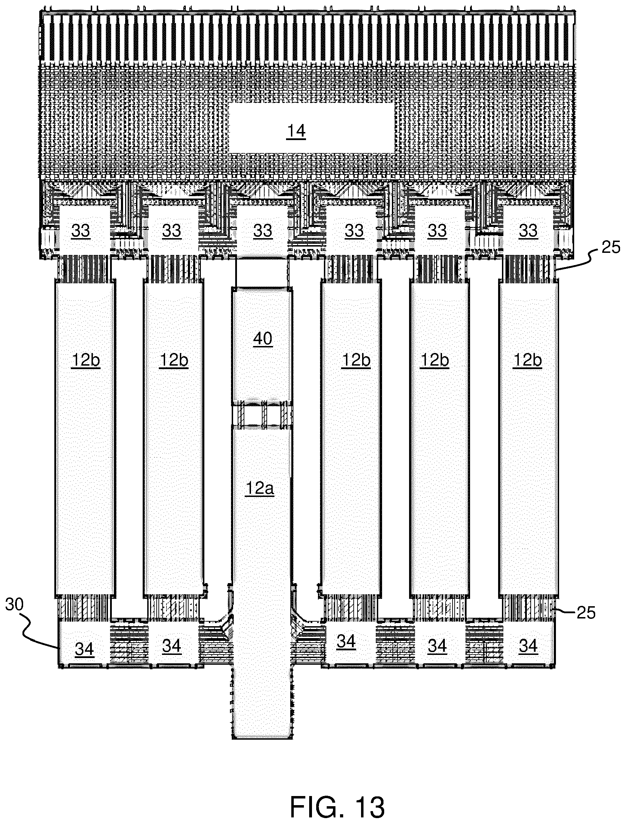

In that case, (at least) two regions can be assigned (and with a suitable area ratio, depending on the needed capacitor values) for these separate capacitors. An example embodiment is described below with reference to Figure 13. The structure of the components of Figure 1 will first be described in more detail.

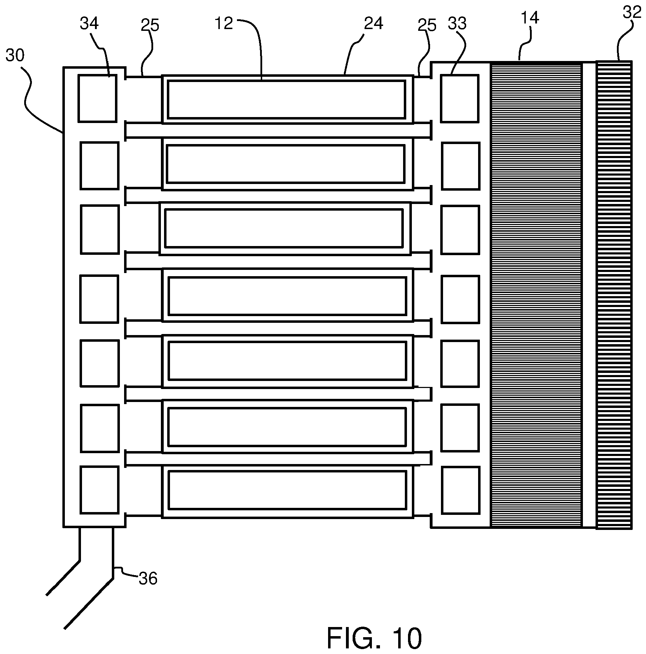

Figure 10 shows an exemplary design before being wrapped around the catheter core 2.

Figure 10 shows the separated integrated circuits 12, and which are mounted on separate support dies 24. F2R connections 25 are conventionally (and in Figure 1) only at the distal end of the support dies 24 of the integrated circuits 12 leading to a distal ring of rigid silicon portions 33. The polyimide of the F2R arrangement is removed between the integrated circuit support dies 24 to allow these to reside more inward relative to the transducer region.

Figure 10 additionally shows an interconnect ring 30 located proximally of the set of integrated circuits 12 and connected to a proximal end of the integrated circuits. The interconnect ring 30 forms a second flexible carrier region. It is connected to the support dies 24 of the integrated circuits by F2R connections 25. A cable or flexible PCB 36 is attached to the interconnect ring.

There are thus rigid silicon die portions 33,34 located at rings (i.e. rings are structures that when folded onto the core 2 form an annular structure around the core) distally and proximally of the ASIC dies, located below the F2R sheet. The rings each contain the silicon dies portions 33, 34 and axially aligned silicon strips (not shown). In this way, the bending of the rings is more predictable. Moreover, the additional silicon areas 33, 34 can be used for integrated capacitors.

The interconnect ring 30 enables signal lines to be redistributed to the integrated circuits 12 at the proximal side of the integrated circuits. The interconnect ring enables extra electrical connections between the integrated circuits as well as a bus connection from the proximal (system) side using a finite number of wires in the cable or flexible PCB 36. The presence of silicon at the interconnect ring 30 facilitates this connection of the cable or flexible PCB 36.

The interconnect ring 30 also provides an improved assembly stability of the ultrasound head (or ultrasound scanner unit), because the integrated circuits 12 are supported at both ends, thereby restricting uncontrolled motions of individual circuits during the assembly process. The interconnect ring may thus be realized in the Flex-to-Rigid (F2R) manufacturing platform, along with the other parts of the ultrasound transducer component.

The interconnect ring 30 also helps to keep the integrated circuits axially aligned to the core 2 during the assembly steps and gives better results in controlling any misalignment.

The interconnect ring 30 for example comprises a single electrical conductor layer (of the F2R system), which is shaped to provide interconnections from the cable or flexible PCB 36 to one or more of the integrated circuits and/or interconnections between the integrated circuits.

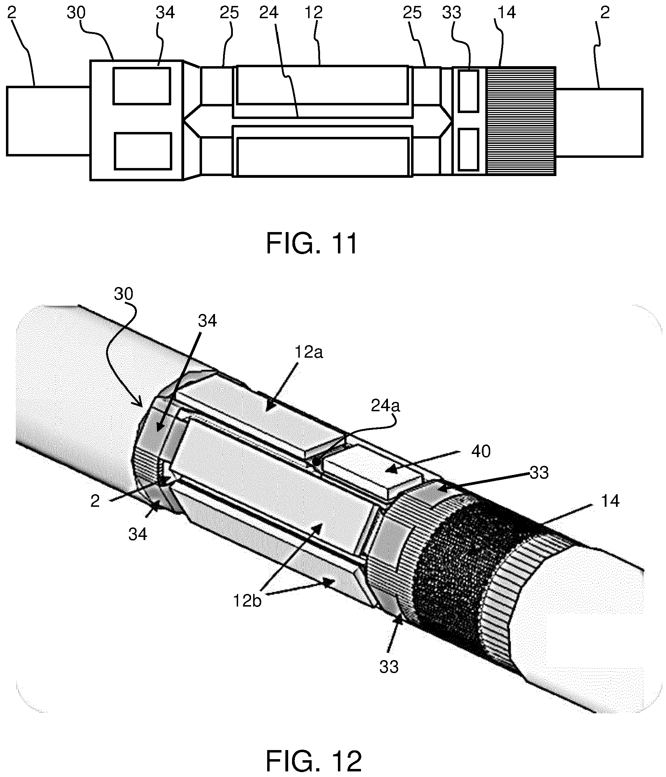

Figure 11 shows the arrangement of Figure 10 provided around the catheter core 2.

Figure 12 shows a variant in which integrated circuits comprise a master ASIC 12a and a set of slave ASICs 12b. The cable connects to the master ASIC 12a and the slave ASICs 12b via the interconnect ring 30, which additionally provides connections between the master ASIC 12a and the slave ASICs 12b.

The support die 24a of the master ASIC 12a also extends beyond the edge of the interconnect ring 30 and thus overlaps a portion of the interconnect ring. It may extend fully over the interconnect ring. This enables space to be provided at the opposite end of the master ASIC 12a. For example, discrete components 40 may be mounted in this space, such as a low voltage capacitor mounted on a separate support die distal to the master ASIC These discrete components for example form part of power supply circuits to the integrated circuits and the ultrasound probe.

Figure 13 shows how this structure may be used for including two types of integrated high voltage capacitors.

The silicon areas 33, the support die for discrete components 40 and a distal part of the support dies beneath the ASICs (not shown in Figure 13) may include capacitors for bias decoupling. The silicon areas 34 and a proximal part of the support dies beneath the ASICs may include decoupling capacitors for the ASIC power supply lines (VDDH). For the best possible electrical performance, the bias decoupling capacitors are placed as close as possible to the cMUT elements and the VDDH decoupling capacitors are placed as close as possible to the ASICs.

A typical bias voltage range is 40 V, and typical VDDH voltage range is 35 V. This arrangement thus has a first capacitor for bias voltage decoupling and a second capacitor for VDDH decoupling.

The invention can be applied to all catheter-like ultrasound imaging products, such as needles, guidewires, using ultrasound or high intensity focused ultrasound (HIFU).

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An ultrasound device comprising:
a shaft (2);
an ultrasound scanner unit (14) at a distal portion (10) of the shaft comprising an array of cMUT ultrasound transducers and one or more capacitors,
wherein the ultrasound transducers each comprise:
a bottom electrode (122) provided over a substrate (112);
a cavity (118) over the bottom electrode (122);
a dielectric layer (114) over the cavity; and
a top electrode (120) over the dielectric layer,
wherein the one or more capacitors comprise:
a bottom electrode (122) provided over the substrate and formed from the same material as the ultrasound transducer bottom electrodes;
a dielectric layer formed from the same material as the ultrasound transducer dielectric layer; and
a top electrode (120) over the dielectric layer and formed from the same material as the ultrasound transducer top electrodes.

2. The device according to claim 1, wherein the one or more capacitors further comprises a sacrificial layer (130) formed over the dielectric layer, and wherein the device further comprises a connection terminal (140) to the sacrificial layer of the one or more capacitors.

3. The device according to claim 1 or 2, wherein one electrode of each ultrasound transducer connects to a ground terminal and the other electrode of each ultrasound transducer connects to a control terminal (150).

4. The device according to claim 3, wherein each ultrasound transducer is part of a transducer circuit which comprises a bias terminal (B) for applying a bias voltage to the ultrasound transducer and a stimulus terminal (S) for applying a drive signal to the ultrasound transducer.

5. The device according to claim 4, wherein the transducer circuit comprises a bias-T circuit comprising, for each ultrasound transducer:
a resistor (R1) between the bias terminal (B) and the control terminal (150); and
the capacitor (C1) between the stimulus terminal (S) and the control terminal.

6. The device according to claim 4, wherein the bias terminal (B) and the stimulus terminal (S) comprise a shared input, and wherein each ultrasound transducer is part of an amplifier circuit, comprising an amplifier (160) and a high pass filter (170), wherein a first input terminal of the amplifier connects to the shared input through the ultrasound transducer, and a second input terminal of the amplifier connects to the shared input through a high pass filter which comprises the capacitor and a resistor.

7. The device according to claim 4, wherein the at least one capacitor comprises a first capacitor for the bias voltage and second capacitor for a supply signal.

8. The device according to any one of claims 1 to 7, comprising:
a first flexible carrier region on which the ultrasound transducers are mounted; and
a set of integrated circuits (12) for controlling the ultrasound transducers, the integrated circuits being located proximally of the ultrasound transducers and connected at a distal end to ultrasound transducers of the array,
wherein the capacitors are formed on the first flexible carrier region.

9. The device according to claim 8, wherein the electrical connection between the integrated circuits and the ultrasound head is implemented with a flex-to-rigid interconnection (25).

10. The device according to claim 8 or 9, further comprising:
a second flexible carrier region (30) which defines an interconnect ring located proximally of the set of integrated circuits and connected to a proximal end of the integrated circuits; and
a cable or flexible PCB (36) attached to the interconnect ring,
wherein the capacitors are formed on the first and second flexible carriers.

11. The ultrasound device as claimed in claim 10, wherein the electrical connection between the interconnect ring and the integrated circuits is implemented with a flex-to-rigid interconnection (25).

12. The ultrasound device as claimed in any one of claims 8 to 11, wherein the integrated circuits (12) are each flip chip bonded to a respective base.

13. The ultrasound device as claimed in any one of claims 8 to 12, wherein the set of integrated circuits (12) are configured in an annular arrangement around the shaft.

14. The ultrasound device as claimed in any one of claims 1 to 13, wherein the ultrasound transducers are configured in an annular array around the shaft.

15. A catheter (40) comprising the ultrasound device as claimed in any one of claims 1 to 14.
